# EUROPÄISCHE PATENTSCHRIFT

(11) **EP 2 928 443 B1**
(45) Veröffentlichungstag und Bekanntmachung des Hinweises auf die Patenterteilung: **26.06.2019**
(21) Anmeldenummer: 13789529.8
(22) Anmeldetag: 07.11.2013
(51) Int. Cl.: A61K 8/34, A61Q 17/04, A61Q 19/00, A61K 8/49

(54) **KOSMETISCHE ODER DERMATOLOGISCHE ZUBEREITUNGEN MIT KOMBINATIONEN AUS 4-N-BUTYLRESORCIN UND TETRAHYDRO-4-METHYL-2-(2-METHYLPROPYL)-2H-PYRAN-4-OL**
COSMETIC OR DERMATOLOGICAL PREPARATIONS WITH COMBINATIONS OF 4-N-BUTYL-RESORCINOL AND TETRAHYDRO-4-METHYL-2-(2-METHYLPROPYL)-2H-PYRAN-4-OL
PRÉPARATIONS COSMÉTIQUES OU DERMATOLOGIQUES COMPORTANT DES COMBINAISONS DE 4-N-BUTYLRÉSORCINE AVEC TETRAHYDRO-4-MÉTHYL-2-(2-MÉTHYLPROPYL)-2H-PYRAN-4-OL

(30) Priorität: 06.12.2012 DE 102012222445
(43) Veröffentlichungstag der Anmeldung: 14.10.2015
(73) Patentinhaber: Beiersdorf AG, 20253 Hamburg (DE)
(72) Erfinder: DINGLER, Christian, 22527 Hamburg (DE); KRÖPKE, Petra, 22869 Schenefeld (DE); HEIL, Nadine, 22605 Hamburg (DE); XU, Yang, Wuhan Hubei 430056 (CN)
(86) Internationale Anmeldenummer: PCT/EP2013/073244
(87) Internationale Veröffentlichungsnummer: WO 2014/086544

(56) Entgegenhaltungen:
- WO-A1-94/27569
- WO-A1-2012/062771
- DE-A1-102006 055 043
- DE-A1-102009 048 970
- DE-A1-102009 048 977
- DE-A1-102010 002 104
- JP-A- 2007 238 573
- US-A1- 2003 180 234
- US-A1- 2003 185 867
- US-A1- 2004 109 832

## Beschreibung

Die vorliegende Erfindung betrifft kosmetische oder dermatologische Zubereitungen mit Kombinationen aus 4-n-Butylresorcin und einem oder mehreren nichtterpenoiden Parfumrohstoffen.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende - pigmentbildende Zellen vorzufinden sind.

Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Homschicht (Comeozyten) transportiert und ruft eine mehr oder weniger ausgeprägte bräunliche bis braun-schwarze Hautfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICA- und DHI-Melanin entstehen über die gemeinsamen Zwischenstufen Dopachinon und Dopachrom. Letzteres wird, teilweise unter Beteiligung weiterer Enzyme, entweder in lndol-5,6-Chinon-Carbonsäure oder in Indol-5,6-Chinon umgesetzt, woraus die beiden genannten Eumelanine entstehen.

Die Entstehung von Phäomelanin läuft unter anderem über die Zwischenprodukte Dopachinon und Cysteinyldopa. Gesteuert wird die Expression der Melanin-synthetisierenden Enzyme durch einen spezifischen Transkriptionsfaktor (microphthalmia- associated transcription factor, MITF). Neben den beschriebenen enzymatischen Prozessen der Melanin-Synthese sind in den Melanosomen noch weitere Proteine für die Melanogenese von Bedeutung. Eine wichtige Rolle scheint hier dem sogenannten p-Protein zuzukommen, wobei die exakte Funktion noch unklar ist.

Neben dem zuvor beschriebenen Prozeß der Melanin-Synthese in den Melanozyten, ist bei der Pigmentierung der Haut auch der Transfer der Melanosomen, deren Verbleib in der Epidermis sowie deren Abbau und der Abbau des Melanins von entscheidender Bedeutung. Es konnte gezeigt werden daß für den Transport der Melanosomen aus den Melanozyten in die Keratinozyten der PAR-2-Rezeptor bedeutsam ist (M. Seiberg et al., 2000, J. Cell. Sci., 113:3093-101).

Ferner haben Größe und Form der Melanosomen Einfluß auf ihre lichtstreuenden Eigenschaften und somit das farbliche Erscheinungsbild der Haut. So findet man bei Schwarzafrikanern verstärkt große spheroidale, einzeln vorliegende Melanosomen, während man bei Kaukasiern eher kleinere, in Gruppen vorkommende Melanosomen vorfindet.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephelides*), genetische Disposition, Fehlpigmentierung der Haut bei der Wundheilung bzw. -vernarbung (postinflammatorische Hyperpigmentierung) oder der Hautalterung (z.B. *Lentigines seniles*).

Nach entzündlichen Reaktionen reagiert das Pigmentierungssystem der Haut mit teilweise entgegengesetzten Reaktionen. Es kann sowohl zu postinflammatorischen Hyper- wie auch Hypopigmentierungen kommen. Postinflammatorische Hypomelanosen treten u. a. häufig in Verbindung mit Atopie, Lupus erythematosus und Psoriasis auf. Die unterschiedlichen Reaktionsformen des Pigmentierungssystems der menschlichen Haut in Folge entzündlicher Erscheinungen sind nur sehr unvollständig verstanden.

Probleme mit postinflammatorischer Hyperpigmentierung treten häufig bei dunkleren Hauttypen auf. Insbesondere bei männlichen Farbigen ist das Problem der *Pseudofollikulitis barbae* bekannt, das mit kosmetisch unerwünschten Fehlpigmentierung einhergeht bzw. diese nach sich zieht. Auch Formen von Melasma, welche insbesondere bei Frauen asiatischer Zugehörigkeit im Gesicht und im Dekolleté - Bereich auftreten, sowie verschiedene Formen der unregelmäßigen Pigmentierung der Haut werden zu den postinflammatorischen Hyperpigmentierungen gezählt. Ferner werden auch dunkle Augenringe als eine Form postinflammatorischen Hyperpigmentierungen angesehen, wobei die zugrunde liegende Entzündung meist subklinisch abläuft.

In vielen Fällen werden derartige postinflammatorische Fehlpigmentierung durch Einwirkung von Sonnenlicht (UV-Licht) noch verstärkt, ohne daß es zu einer UV-induzierten Entzündung (Sonnenbrand) kommt.

Es sind Wirkstoffe und Zubereitungen bekannt, welche der Hautpigmentierung entgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Von Albert Kligman et al. wurde eine sogenannte Triformula entwickelt, die eine Kombination aus 0.1% Tretinoin, 5.0% Hydrochinon, 0.1% Dexamethason darstellt (A. Kligman, 1975, Arch. Dermatol., 111:40-48). Allerdings ist auch diese Formulierung wegen möglicher irreversibler Veränderungen im Pigmentierungssystem der Haut sehr umstritten.

Ferner finden hautschälende Methoden (chemische und mechanische "Peelings") Anwendung, die jedoch häufig entzündliche Reaktionen nach sich ziehen und aufgrund danach eintretender postinflammatorischer Hyperpigmentierungen sogar zu stärkerer statt verminderter Pigmentierung führen können. All diese gängigen Verfahren, die auch zur Behandlung von postinflammatorischen Hypergigmentierungen angewendet werden, zeichnen sich durch entscheidende Nebenwirkungen aus.

Ziel der nachfolgenden Erfindung war es also, dem nachteiligen Stand der Technik Abhilfe zu verschaffen.

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (dispere oder innere Phase), während die andere Flüssigkeit eine kontinuierliche (kohärente oder innere Phase) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Neuere Erkenntnisse führten in letzter Zeit zu einem besseren Verständnis praxisrelevanter kosmetischer Emulsionen. Dabei geht man davon aus, daß die im Überschuß eingesetzten Emulgatorgemische lamellare flüssigkristalline Phasen bzw. kristalline Gelphasen ausbilden. In der Gelnetzwerktheorie werden Stabilität und physikochemische Eigenschaften solcher Emulsionen auf die Ausbildung von viskoelastischen Gelnetzwerden zurückgeführt.

Kosmetische Zubereitungen mit 4-n-Butylresorcin sind bekannt, beispielsweise aus der EP 1 490 017.

4-n-Butylresorcin wird auch Rucinol genannt. Es hemmt die Melaninproduktion, indem es zwei für die Melaninsynthese (Melanogenese) erforderliche Enzyme hemmt, die Tyrosinase und die 5,6-Dihydroxyindol-2-carbonsäure-Oxidase. Es wird zunächst die Melaninproduktion gehemmt, dann die Produktion des schwarzen Melanins, das für die intensive Färbung der Pigmentflecken verantwortlich ist, blockiert.

Obwohl die Verwendung von 4-n-Butylresorcin kosmetisch zweifellos vorteilhaft wäre, sind kosmetische Zubereitungen mit einem Gehalt an 4-n-Butylresorcin schwierig zu stabilisieren, da dieses empfindlich gegen Oxidation ist. Dies macht sich durch verschiedene Erscheinungen bemerkbar, unter anderem die, daß sich Zubereitungen mit 4-n-Butylresorcin schnell bräunlich verfärben.

Es war also eine Aufgabe der vorliegenden Erfindung, diesem Übelstande abzuhelfen.

Es war überraschend und für den Fachmann nicht vorhersehbar, daß Übelstände des Standes der Technik beseitigt werden durch kosmetische oder dermatologische Zubereitungen mit Kombinationen aus 4-n-Butylresorcin und Tetrahydro-4-methyl-2-(2-methylpropyl)-2H-pyran-4-ol 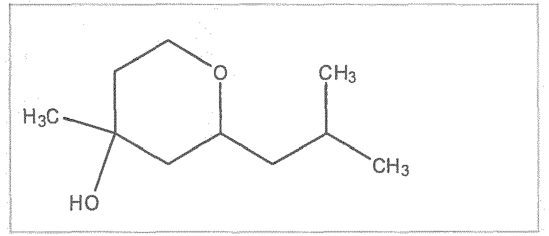 (CAS-Nummer 63500-71-0).

Die DE 10 2009 048 977 A1 offenbart stabilisiertes 4-n-Butylresorcin, konnte aber nicht den Weg zur vorliegenden Erfindung weisen.

4-n-Butylresorcin ist durch die chemische Struktur 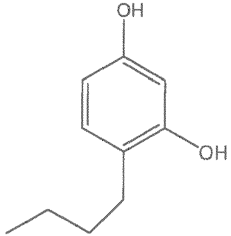 gekennzeichnet.

Bevorzugt enthalten kosmetische oder dermatologische Zubereitungen gemäß der Erfindung 0,001 - 10 Gew.-%, besonders bevorzugt 0,01 - 1 Gew.-%, an 4-n-Butylresorcin, bezogen auf die Gesamtzusammensetzung der Zubereitungen.

Eine besonders bevorzugte Zusammensetzung im Sinne der Vorliegenden Erfindung wird verkörpert durch Zubereitungen, enthaltend 0,001 - 10 Gew.-%, besonders bevorzugt 0,01 - 5 Gew.-%, ganz besonders bevorzugt 0,1 - 1 Gew.-% an Tetrahydro-4-methyl-2-(2-methylpropyl)-2H-pyran-4-ol.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an üblichen Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung. Besonders vorteilhaft sind Natriumdisulfit und/oder Diethylhexylsyringylidenmalonat, insbesondere eine Mischung beider Substanzen, die unter der Handelsbezeichnung Oxynex ST liquid verkauft wird.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die Lichtschutzformulierungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**O/W-Emulsion**

| | **1** | **2** | **3** | **4** | **5** |
|---|---|---|---|---|---|
| Polyethylenglycol(21)stearylether | 1 | -- | 2,5 | 2 | 1,5 |
| Polyethylenglycol(2)stearylether | 1 | -- | 5,5 | 3 | 7,5 |
| Cetearylglucosid | --- | 8 | --- | --- | --- |
| cyclisches Silikonöl | 12,5 | 15 | 28,0 | 25,0 | 17,5 |
| lineares Silikonöl | 5,0 | 13,0 | 5,0 | 12,0 | 15,0 |
| Behenylalkohol | 3 | 2 | --- | 1 | --- |
| Stearylalkohol | 3 | 2 | --- | 2 | --- |
| Carbomer | 0,3 | 0,45 | 0,1 | 0,05 | 1,0 |
| hydriertes Polyisobuten | 0,5 | 0,75 | 1,0 | 2,0 | 0,25 |
| Glitterpigmente | 5 | 10 | 15 | 20 | 7,5 |
| Octyldodecanol | 0,5 | 1,0 | 0,75 | 3,0 | 0,25 |
| Glycerin | 5 | 10 | 15 | 3 | 7,5 |
| Panthenol | 0,5 | 1,0 | 0,75 | 0,25 | 0,1 |
| Methylparaben | 0,4 | 0,1 | 0,05 | 0,3 | 0,4 |
| Propylparaben | 0,3 | 0,4 | 0,25 | 0,15 | --- |
| modifizierte Stärke | 0,5 | --- | --- | 0,15 | -- |
| Erfindungsgemäße Parfummischung 1 | 0,03 | 2 | --- | --- | --- |
| Erfindungsgemäße Parfummischung 2 | --- | --- | 0,01 | --- | 1 |
| Erfindungsgemäße Parfummischung 3 | --- | --- | --- | 0,5 | --- |
| Ggf. Weitere Parfum Rohstoffe | q,s, | q,s, | q,s, | q,s, | q,s, |
| Wasser | ad 100 | ad 100 | ad 100 | ad 100 | ad 100 |

**O/WN-Emulsion**

| | **6** | **7** | **8** | **9** | **10** |
|---|---|---|---|---|---|
| Cetylstearylalkohol + PEG-40 Rizinusöl + Natriumcetylstearylsulfat | 2,8 | 3,1 | 2,1 | 0,75 | 5,5 |
| Cetylakohol | 1,0 | 1,0 | 5 | 2,2 | -- |
| Glycerylstearat, selbstemulgierend | 0,7 | 0,75 | 2,25 | --- | 2,5 |
| Caprylic/Capric Triglycerid + Algenextrakt | 0,1 | 0,1 | 0,50 | 0,25 | 0,5 |
| Titandioxid Cl 77891 | 0,2 | --- | 0,5 | 1 | -- |
| Diethylhexylsyringylidenmalonat | 0,1 | 0,2 | 0,3 | 0,4 | 0,5 |
| Rucinol | 0,3 | 0,4 | 2 | 1 | 0,2 |
| lineares Silikonöl | 2,0 | 5,0 | 1,0 | 1,5 | 0,75 |
| Tocopherolacetat | 0,06 | 0,08 | 0,1 | 0,005 | 0,2 |
| Cetylstearylalkohol | --- | --- | 1 | 1 | 0,2 |
| Tocopherolacetat | 0,5 | 0,5 | 0,75 | 0,25 | 0,1 |
| EDTA, Trinatriumsalz | 0,2 | 0,5 | 1,0 | 0,2 | 0,25 |
| Glycerylglucosid | 0,01 | 0,02 | 0,75 | 0,25 | 0,1 |
| Natriumpolyacrylat | 0,3 | 0,35 | 0,15 | 0,1 | 0,05 |
| hydrolysierter Perlenextrakt | 0,05 | 0,75 | 0,1 | 1,5 | 1,0 |
| Xanthangum | 0,2 | 0,3 | 0,05 | 0,3 | 0,4 |
| Acrylamid/Ammoniumacrylatcopolymer + Polyisobuten + Polysorbat-20 | 0,3 | 0,2 | 0,25 | 0,15 | --- |
| Ethanol | 2,0 | 0,5 | --- | 0,15 | 5,0 |
| Ginsengwurzelextrakt | 0,03 | 0,05 | -- | 0,5 | 0,75 |
| Lotusextrakt | 0,1 | 0,25 | --- | 0,05 | 1,0 |
| Süssholzwurzelextrakt | 0,5 | 0,3 | 1,0 | 0,5 | --- |
| Glycerin | 3,0 | 7,5 | 1,5 | 0,75 | 5,0 |
| Natriumbisulfit | 0,15 | 0,25 | 0,05 | 0,75 | 0,05 |
| Distarchphosphat | 3,0 | 0,25 | 1,5 | 0,75 | 5,0 |
| Methylisothiazolinon | 0,09 | 0,08 | 0,07 | 0,06 | 0,05 |
| Natriumascorbylphosphat | 0,01 | 0,75 | 0,15 | 0,75 | 0,05 |
| Erfindungsgemäße Parfummischung 1 | --- | --- | --- | 10 | 0,1 |
| Erfindungsgemäße Parfummischung 2 | --- | 0,01 | --- | --- | --- |
| Erfindungsgemäße Parfummischung 3 | 0,05 | --- | 0,9 | --- | --- |
| Ggf. Weitere Parfum Rohstoffe | q,s, | q,s, | q,s, | q,s, | q,s, |
| Wasser | ad 100 | ad 100 | ad 100 | ad 100 | ad 100 |

**Parfümmischungen**

| CAS-Nummer | Parfümmischung 1 | Parfümmischung 2 | Parfümmischung 3 |
|---|---|---|---|
| 63500-71-0 | 20 | 10 | 15 |
| 55066-48-3 | 10 | 5 | 10 |
| 60-12-8 | 10 | 5 | 2 |
| 28219-61-6 | 10 | 5 | 0 |
| 65405-77-8 | 5 | 1 | 1 |
| 1205-17-0 | 5 | 1 | 5 |
| 103-95-7 | 5 | 1 | 4 |
| 81782-77-6 | 5 | 1 | 1 |
| 123-11-5 | 5 | 1 | 1 |
| 2550-26-7 | 5 | 1 | 5 |
| 88-41-5 | 5 | 1 | 4 |
| 67801-20-1 | 5 | 1 | 1 |
| 25265-71-8 | ad 100 | ad 100 | ad 100 |
| | 90 | 33 | 49 |

## Patentansprüche

1. Kosmetische oder dermatologische Zubereitungen mit Kombinationen aus 4-n-Butylresorcin und Tetrahydro-4-methyl-2-(2-methylpropyl)-2H-pyran-4-ol 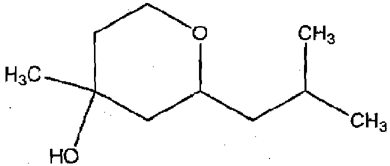 (CAS-Nummer 63500-71-0).

2. Zubereitungen nach Anspruch 1, **dadurch gekennzeichnet, daß** sie in Form von Emulsionen vorliegen.

3. Zubereitung nach Anspruch 1 oder 2, **dadurch gekennzeichnet, daß** die Zubereitun gen 0,001 - 10 Gew.-%, besonders bevorzugt 0,01 - 1 Gew.-%, an 4-n-Butylresorcin enthalten, bezogen auf die Gesamtzusammensetzung der Zubereitungen.

4. Zubereitung nach einem der vorstehenden Ansprüche, **dadurch gekennzeichnet, daß** die Zubereitungen 0,001 - 10 Gew.-%, besonders bevorzugt 0,01 - 5 Gew.-%, ganz besonders bevorzugt 0,1 - 1 Gew.-% an einem oder mehreren nichtterpenoiden Par fumrohstoffen enthalten, bezogen auf die Gesamtzusammensetzung der Zubereitungen.

## Claims

1. Cosmetic or dermatological preparations with combinations of 4-n-butylresorcinol and tetrahydro-4-methyl-2-(2-methylpropyl)-2H-pyran-4-ol 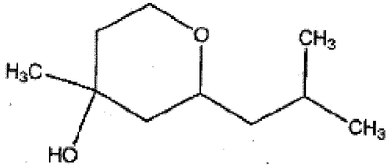 (CAS Number 63500-71-0).

2. Preparations according to Claim 1, **characterized in that** said preparations are in the form of emulsions.

3. Preparation according to Claim 1 or 2, **characterized in that** the preparations comprise 0.001 - 10% by weight, particularly preferably 0.01 to 1% by weight, of 4-n-butylresorcinol, based on the total composition of the preparations.

4. Preparation according to any of the preceding claims, **characterized in that** the preparations comprise 0.001 - 10% by weight, particularly preferably 0.01 to 5% by weight, especially preferably 0.1 - 1% by weight of one or more non-terpenoid perfume raw materials, based on the total composition of the preparations.

## Revendications

1. Préparations cosmétiques ou dermatologiques contenant des combinaisons de 4-n-butylrésorcine et de tétrahydro-4-méthyl-2-(2-méthylpropyl)-2H-pyran-4-ol 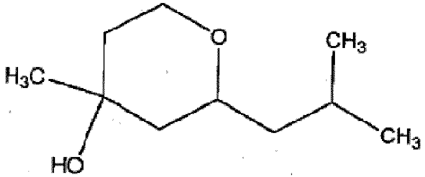 (numéro CAS 63500-71-0)

2. Préparations selon la revendication 1, **caractérisées en ce qu'**elles se présentent sous la forme d'émulsions.

3. Préparation selon la revendication 1 ou 2, **caractérisée en ce que** les préparations contiennent 0,001 à 10% en poids, de manière particulièrement préférée 0,01 à 1 % en poids, de 4-n-butylrésorcine, par rapport à la composition totale des préparations.

4. Préparation selon l'une quelconque des revendications précédentes, **caractérisée en ce que** les préparations contiennent 0,001 à 10 % en poids, de manière particulièrement préférée 0,01 à 5 % en poids, de manière tout particulièrement préférée 0,1 à 1 % en poids, d'une ou de plusieurs matières premières parfumées non terpénoïdes, par rapport à la composition totale des préparations.
